# EUROPEAN PATENT APPLICATION

(11) **EP 1 982 994 A2**
(43) Date of publication of application: **22.10.2008**
(21) Application number: 06828471.0
(22) Date of filing: 28.12.2006
(51) Int. Cl.: C07K 14/22, A61K 38/16, G01N 33/68

(54) **CARBOHYDRATE-MIMETIC PEPTIDES AND USE THEREOF IN PHARMACEUTICAL FORMULATIONS**

(30) Priority: 29.12.2005 CU 2832005
(71) Applicant: CENTRO DE INGENIERIA GENETICA Y BIOTECNOLOGIA, Ciudad de La Habana 10600 (CU)
(72) Inventor: MENÉNDEZ MEDINA, Tamara, Ciudad De La Habana 11300 (CU); CRUZ LEAL, Yoelys, Ciudad De La Habana 10600 (CU); REYES ACOSTA, Osvaldo, Ciudad De La Habana 10600 (CU); GARAY PÉREZ, Hilda, Elisa, Ciudad De La Habana 10600 (CU); GUILLÉN NIETO, Gerardo, Enrique, Ciudad De La Habana 10600 (CU); COIZEAU RODRÍGUEZ, Edelgis, Ciudad De La Habana 10600 (CU); SANTIAGO VISPO, Nelson, Francisco, Ciudad De La Habana 10600 (CU); CHINEA SANTIAGO, Glay, Ciudad De La Habana 10600 (CU)
(74) Representative: Hatzmann, Martin
(86) International application number: PCT/CU2006/000020
(87) International publication number: WO 2007/073706

(57) **Abstract**

The present invention is related with the pharmaceutical industry and the biotechnology, specifically with vaccine antigens applied against bacterial, viral, cancerous or another origin diseases. Development of pharmaceuticals formulations able to protect or to increase the protective spectrum of already developed vaccines and to extend it against several pathogens, using as target for the immune response, carbohydrates composed of repeated units of (α 2-8) linked N-replaced neuraminic acid. Several peptides were isolated and identified as molecular mimetics of the capsular polysaccharide form serogroup B of *Neisseria meningitidis,* which is composed of repeated units of (α 2-8) linked N-acetyl neuraminic acid. The immunogenicity of peptides was evaluated in animal biomodels, demonstrating their value as antigens able to induce the production of antibodies able of specifically recognize the bacterium *N. meningitidis* from serogroup B and of showing bactericidal activity against the same one. The resulting formulations of are applicable in the pharmaceutical industry as vaccine formulations for human use.

SEQUENCE LISTING

< 110 > CENTRO DE INGENIERIA GENETICA Y BIOTECNOLOGIA

< 120 > CARBOHYDRATE-MIMETIC PEPTIDES AND USE THEREOF IN PHARMACEUTICAL
FORMULATIONS

< 130 > P85154NO00

< 150 > PCT/CU2006/000020
< 151 > 2006-12-28

< 150 > CU 2005-0283
< 151 > 2005-12-29

< 160 > 29

< 170 > PatentIn Ver. 2.1

< 210 > 1
< 211 > 45
< 212 > DNA
< 213 > Artificial Sequence

< 220 >
< 223 > Description of the Artificial Sequence:
Combination at random of 15 amino acids

< 220 >
< 221 > gene
< 222 > (1)..(45)
< 223 > DNA Sequence that codifies for the peptide
NMGBPS1

< 400 > 1
aggggggaca agagcaggcc gcccgtgtgg tacgtggagg gggag 45

< 210 > 2
< 211 > 45
< 212 > DNA
< 213 > Artificial Sequence

< 220 >
< 223 > Description of the Artificial Sequence:
Combination at random of 15 amino acids

< 220 >
< 221 > gene
< 222 > (1)..(45)
< 223 > DNA Sequence that codifies for the peptide
NMGBPS2

< 400 > 2
ccggcggggc ggcagcgcgc caggcccgcg acggtgcccg agggg 45

< 210 > 3
< 211 > 45
< 212 > DNA
< 213 > Artificial Sequence

< 220 >
< 223 > Description of the Artificial Sequence:
Combination at random of 15 amino acids

< 220 >
< 221 > gene

< 222 > (1)..(45)
< 223 > DNA Sequence that codifies for the peptide
        NMGBPS3

< 400 > 3
cccgagtggt ggctcgagga gccgccgttc atcagctcgg gcaag                45


< 210 > 4
< 211 > 45
< 212 > DNA
< 213 > Artificial Sequence

< 220 >
< 223 > Description of the Artificial Sequence:
        Combination at random of 15 amino acids

< 220 >
< 221 > gene
< 222 > (1)..(45)
< 223 > DNA Sequence that codifies for the peptide
        NMGBPS4

< 400 > 4
ccggtccagc gggggcgtg gaggtggccg gtcgtgacga acgag        45


< 210 > 5
< 211 > 15
< 212 > PRT
< 213 > Artificial Sequence

< 220 >
< 223 > Description of the Artificial Sequence:
        Combination at random of 15 amino acids

< 220 >
< 221 > PEPTIDE
< 222 > (1)..(15)
< 223 > Peptide NMGBPS1

< 400 > 5
Arg Gly Asp Lys Ser Arg Pro Pro Val Trp Tyr Val Glu Gly Glu
1               5                   10                  15


< 210 > 6
< 211 > 15
< 212 > PRT
< 213 > Artificial Sequence

< 220 >
< 223 > Description of the Artificial Sequence:
        Combination at random of 15 amino acids

< 220 >
< 221 > PEPTIDE
< 222 > (1)..(15)
< 223 > Peptide NMGBPS2

< 400 > 6
Pro Ala Gly Arg Gln Arg Ala Arg Pro Ala Thr Val Pro Glu Gly
1               5                   10                  15


< 210 > 7
< 211 > 15
< 212 > PRT

<213> Artificial Sequence

<220>
<223> Description of the Artificial Sequence:
      Combination at random of 15 amino acids

<220>
<221> PEPTIDE
<222> (1)..(15)
<223> Peptide NMGBPS3

<400> 7
Pro Glu Trp Trp Leu Glu Glu Pro Pro Phe Ile Ser Ser Gly Lys
1               5                   10                  15


<210> 8
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS4

<400> 8
Pro Val Gln Arg Gly Ala Trp Arg Trp Pro Val Val Thr Asn Glu
1               5                   10                  15


<210> 9
<211> 15
<212> PRT
<213> Artificial Sequence

<220>
<221> PEPTIDE
<222> (1)..(15)
<223> Peptide NMGBPS5

<220>
<223> Description of the Artificial Sequence:
      Combination at random of 15 amino acids

<400> 9
Arg Gly Asp Lys Ser Arg Ser Ser Ser Trp Tyr Val Glu Gly Glu
1               5                   10                  15


<210> 10
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS6

<400> 10
Arg Gly Asp Lys Ser Arg Ala Ala Val Trp Tyr Ala Glu Gly Glu
1               5                   10                  15

<210> 11
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS7

<400> 11
Arg Gly Asp Lys Ser Arg Ala Ala Val Trp Tyr Val Glu Gly Glu
1               5                   10                  15

<210> 12
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS8

<400> 12
Arg Gly Asp Lys Ser Arg Ala Ala Val Trp Tyr Ser Glu Gly Glu
1               5                   10                  15

<210> 13
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS9

<400> 13
Arg Gly Asp Lys Ser Arg Ala Ala Val Trp Tyr Glu Glu Gly Glu
1               5                   10                  15

<210> 14
<211> 15
<212> PRT
<213> Artificial Sequence

<220>

<223> Description of the Artificial Sequence:
Combination at random of 15 amino acids

<220>
<221> PEPTIDE
<222> (1)..(15)
<223> Peptide NMGBPS10

<400> 14
Arg Gly Asp Lys Ser Arg Ala Ala Val Trp Glu Val Glu Gly Glu
1               5                   10                  15

<210> 15
<211> 15
<212> PRT
<213> Artificial Sequence

<220>
<223> Description of the Artificial Sequence:
Combination at random of 15 amino acids

<220>
<221> PEPTIDE
<222> (1)..(15)
<223> Peptide NMGBPS11

<400> 15
Arg Gly Asp Lys Ser Arg Ala Ala Val Trp Ile Val Glu Gly Glu
1               5                   10                  15

<210> 16
<211> 15
<212> PRT
<213> Artificial Sequence

<220>
<223> Description of the Artificial Sequence:
Combination at random of 15 amino acids

<220>
<221> PEPTIDE
<222> (1)..(15)
<223> Peptide NMGBPS12

<400> 16
Arg Gly Asp Lys Ser Arg Pro Pro Val Trp Tyr Ala Glu Gly Glu
1               5                   10                  15

<210> 17
<211> 15
<212> PRT
<213> Artificial Sequence

<220>
<223> Description of the Artificial Sequence:
Combination at random of 15 amino acids

<220>
<221> PEPTIDE
<222> (1)..(15)
<223> Peptide NMGBPS13

<400> 17
Arg Gly Asp Lys Ser Arg Pro Pro Val Trp Tyr Ser Glu Gly Glu
1               5                   10                  15

< 210 > 18
< 211 > 15
< 212 > PRT
< 213 > Artificial Sequence

< 220 >
< 223 > Description of the Artificial Sequence:
Combination at random of 15 amino acids

< 220 >
< 221 > PEPTIDE
< 222 > (1)..(15)
< 223 > Peptide NMGBPS14

< 400 > 18
Arg Gly Asp Lys Ser Arg Pro Pro Val Trp Tyr Glu Glu Gly Glu
1               5                   10                  15

< 210 > 19
< 211 > 15
< 212 > PRT
< 213 > Artificial Sequence

< 220 >
< 223 > Description of the Artificial Sequence:
Combination at random of 15 amino acids

< 220 >
< 221 > PEPTIDE
< 222 > (1)..(15)
< 223 > Peptide NMGBPS15

< 400 > 19
Arg Gly Asp Lys Ser Arg Pro Ala Val Trp Tyr Val Glu Gly Glu
1               5                   10                  15

< 210 > 20
< 211 > 15
< 212 > PRT
< 213 > Artificial Sequence

< 220 >
< 223 > Description of the Artificial Sequence:
Combination at random of 15 amino acids

< 220 >
< 221 > PEPTIDE
< 222 > (1)..(15)
< 223 > Peptide NMGBPS16

< 400 > 20
Arg Gly Asp Lys Ser Arg Pro Ala Val Trp Tyr Ala Glu Gly Glu
1               5                   10                  15

< 210 > 21
< 211 > 15
< 212 > PRT
< 213 > Artificial Sequence

< 220 >
< 223 > Description of the Artificial Sequence:
Combination at random of 15 amino acids

< 220 >
< 221 > PEPTIDE
< 222 > (1)..(15)
< 223 > Peptide NMGBPS17

< 400 > 21
Arg Gly Asp Lys Ser Arg Pro Ala Val Trp Tyr Ser Glu Gly Glu
1               5                   10                  15

< 210 > 22
< 211 > 15
< 212 > PRT
< 213 > Artificial Sequence

< 220 >
< 223 > Description of the Artificial Sequence:
        Combination at random of 15 amino acids

< 220 >
< 221 > PEPTIDE
< 222 > (1)..(15)
< 223 > Peptide NMGBPS18

< 400 > 22
Arg Gly Asp Lys Ser Arg Pro Ala Val Trp Tyr Glu Glu Gly Glu
1               5                   10                  15

< 210 > 23
< 211 > 15
< 212 > PRT
< 213 > Artificial Sequence

< 220 >
< 223 > Description of the Artificial Sequence:
        Combination at random of 15 amino acids

< 220 >
< 221 > PEPTIDE
< 222 > (1)..(15)
< 223 > Peptide NMGBPS19

< 400 > 23
Arg Gly Asp Lys Ser Arg Ala Pro Val Trp Tyr Val Glu Gly Glu
1               5                   10                  15

< 210 > 24
< 211 > 15
< 212 > PRT
< 213 > Artificial Sequence

< 220 >
< 223 > Description of the Artificial Sequence:
        Combination at random of 15 amino acids

< 220 >
< 221 > PEPTIDE
< 222 > (1)..(15)
< 223 > Peptide NMGBPS20

< 400 > 24
Arg Gly Asp Lys Ser Arg Ala Pro Val Trp Tyr Ala Glu Gly Glu
1               5                   10                  15

< 210 > 25

<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS21

<400> 25
Arg Gly Asp Lys Ser Arg Ala Pro Val Trp Tyr Ser Glu Gly Glu
1               5                   10                  15

<210> 26
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS22

<400> 26
Arg Gly Asp Lys Ser Arg Ala Pro Val Trp Tyr Glu Glu Gly Glu
1               5                   10                  15

<210> 27
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS23

<400> 27
Arg Gly Asp Lys Ser Arg Pro Pro Val Phe Tyr Val Glu Gly Glu
1               5                   10                  15

<210> 28
<211> 15
<212> PRT
<213> Artificial Sequence

<220>
<223> Description of the Artificial Sequence:
      Combination at random of 15 amino acids

<220>
<221> PEPTIDE
<222> (1)..(15)

<223> Peptide NMGBPS24

<400> 28
Arg Gly Asp Lys Ser Arg Pro Ala Val Phe Tyr Val Glu Gly Glu
1               5                   10                  15


<210> 29
<211> 15
<212> PRT
<213> Artificial Sequence

<220>
<223> Description of the Artificial Sequence:
      Combination at random of 15 amino acids

<220>
<221> PEPTIDE
<222> (1)..(15)
<223> Peptide NMGBPS25

<400> 29
Arg Gly Asp Lys Ser Arg Ala Pro Val Phe Tyr Val Glu Gly Glu
1               5                   10                  15

## Description

### Field of the invention

The present invention is related with the field of medicine, particularly with the development of new vaccine formulations for preventive or therapeutic applications which allow induction or increase of the immune response against specific antigens to fight diseases of diverse origin.

### Background of the invention

Carbohydrates with chemical structure consisting of repeated units of (α 2-8)-linked N-replaced neuraminic acid occur as components of the polysaccharide capsule of the bacterium *N. meningitidis* of serogroup B. Particularly, the capsule of this bacterium is composed of repeated units of (α 2-8) linked N-acetyl neuraminic acid (Bhattacharjee A. K. et al. 1975. Structural determination of the sialic acid polysaccharide antigens of Neisseria meningitidis serogroups B and C with carbon 13 nuclear magnetic resonance. J. Biol. Chem. 250: 1926-1932). *Neisseria meningitidis* is a Gram negative bacterium whose only host are humans and constitutes the causal agent of the meningococcal disease. Usually, this bacterium is found in the natural transient microbial population colonizing the upper respiratory tract of healthy individuals known referred to as carriers. This is the commonest source of the isolates.

Children of less than 2 years of age are the most susceptible to contract the meningococcal meningitis, which is the most frequent clinical complication of the meningococcal disease. Nevertheless, teen-agers and adults can also be affected.

Untreated meningococcal disease is fatal in most of the affected individuals, an outcome that could be prevented by vaccination, which even prevent such early stages as bacterial colonization.

*N. meningitidis* clinical isolates are usually capsulated. Structural differences in the capsule of isolates cause antigenic differences and are the basis for the classification of the bacteria into several serogroups (Rosenstein N. E. and Perkins B. A. 2000. Update on Haemophilus influenzae serotype b and meningococcal vaccines. Pediatr. Clin. North. Am., 47: 337- 352).

Serogroups A, B, C, Y and W-135 are the most important from a clinical point of view among the 13 *N. meningitidis* serogroups reported so far. Serogroup A is the main cause of the disease epidemics in sub-Sahara Africa. Serogroups B and C are associated with most of the cases occurring in the developed countries. Serogroups Y and W-135 are responsible for most of the remaining cases of disease and infection reported in some regions of the United States of America, with a significant increase in the last years (Rosenstein N. et al. 2001. Meningococcal disease. N. Engl. J. Med, 344, 1378-1388).

Serogroup B is responsible for 50-70% of bacterial meningitis cases reported in infants and children, it can cause fatal sepsis in infected adolescents (Romero J.D. and Outschoorn I.M. 1997. The immune response to the capsular polysaccharide of Neisseria meningitidis group B. Zentralbl. Bakteriol. 285: 331-340). One of the strategies tried to fight disease caused by the meningococcal serogroup B has been the use of blebs released from the bacterial outer membrane during the exponential growth phase as immunogen. These blebs are known as outer membrane vesicles (OMV). To be used in vaccines, the vesicles are purified, with the lipopolysaccharides (LPS) being removed by detergent treatments. The antigens in these vesicles are mainly bacterial outer membrane proteins (Frasch C.E. et al. 1988. Antibody response of adults to an aluminum hydroxide-adsorbed Neisseria meningitidis serotype 2b protein-group B polysaccharide vaccine. J. Infect. Dis. 158: 710-718). Several vaccines based on OMV have been assayed in clinical trials (Rosenqvist E. et al. 1991. Human antibody responses after vaccination with the Norwegian group B meningococcal outer membrane vesicle vaccine: results from ELISA studies. NIPH Ann. 14: 169-79; Sierra G.V. et al. 1991. Vaccine against group B Neisseria meningitidis: protection trial and mass vaccination results in Cuba. NIPH Ann. 14: 195-207). The Cuban vaccine, commercially known as VA-MENGOC-BC, based on a strain of serological classification B:4:P1.19,15, the most frequent isolate from cases in Cuba, showed an efficacy of 83% in field clinical trials. The incidence of meningococcal disease decreased from 14.4 infected people per 100 000 inhabitants in 1983 to 0.45 cases per 100 000 inhabitants in 1994 as a result of the massive application of the vaccine in Cuba (Sierra G. V. et al. 1991. Vaccine against group B Neisseria meningitidis: protection trial and mass vaccination results in Cuba. NIPH Ann. 14: 195-207; Diaz R.J. and Outschoorn I. M. 1994. Current status of meningococcal group B vaccine candidates: capsular or noncapsular? Clin. Microbiol. Rev. 7: 559-575). The application of this vaccine in Sao Paulo, Brazil, from 1989 to 1990, controlled the epidemic outbreaks in this region (de Moraes J.C. et al. 1992. Protective efficacy of a serogroup B meningococcal vaccine in Sao Paulo, Brazil. Lancet 340:1074- 1078). However, these vaccines are produced from one strain only, causing protection to be serotype or subtype-specific, limited mainly to the source strain. On the other hand, the circulation of *N*. *meningitidis* serogroup B strains varies temporary and geographically. The use of this type of vaccines is therefore limited to isolated geographic regions where the circulation of strains is limited or to regions with epidemic outbreaks where the strain or the strains responsible for the outbreaks are well characterized and an specific vaccine is produced to stop these epidemic (Holst J. et al. 2005. The concept of Tailor-made, protein-based, outer membrane vesicle vaccine against meningococcal disease. Vaccine 23: 2202-2205). The simple combination of different preparations of OMV from several strains is not an effective alternative, since it could attain high levels of LPS content per dose of vaccines with risks of causing side effects (Diaz R.J. and Outschoorn I. M. 1994. Current status of meningococcal group B vaccine candidates: capsular or noncapsular? Clin. Microbiol. Rev. 7: 559-575).

Vaccines based on the capsular polysaccharides purified from cells of *N. meningitides* have been able to protect against meningococcal disease caused by the serogroups A, C, Y and W135. These vaccines are capable to induce bactericidal and protective antibodies in adults and effective in the control of epidemic outbreaks (Rosenstein N. et al. 2001. Menningococcal disease. N. Engl. J. Med, 344: 1378-1388). A new generation of vaccines, based on the polysaccharide purified from serogroup C bacterium conjugated to carrier proteins, is available in the market. These vaccines demonstrated their capacity of induction of bactericidal and protective antibodies, not only in adults but also in small children in field clinical trials in England (Miller E. et al. 2002. Planning, registration and implementation of an immunisation campaign against meningococcal serogroup C disease in the UK: a success story. Vaccine 20; s58-s67). Furthermoree, the conjugated vaccines allow to overcome the main difficulties associated with the use of plain polysaccharides as immunogens, such as the induction of tymus-independent and short-duration immune response, the absence of stimulation of memory immune response with maturation of affinity. Also it as been described the induction of an hyporesponsiveness phenomenon, well documented in the case of serogroup C, in individuals vaccinated repeatedly with these vaccines, for example in the regions of high incidence of the disease (Jennings H. and Lugowski C. 1981. Immunochemistry of groups A, B and C meningococcal polysaccharide-te tan us toxoid conjugates. J Immunol 127: 1011-1018; Gold R. and Lepow M. L. 1975. Clinical evaluation of group A and C meningococcal polysaccharide vaccines in infants. J. Clin Invest 56: 1536-47; Borrow R. et al. 2001. Influence of prior meningococcal C polysaccharide vaccination on the response and generation of memory after meningococcal C conjugate vaccination in young children. J Infect Dis 184: 377-380). Several conjugate vaccines against the serogroup A are undergoing clinical studies that have demonstrated to be save, immunogenic and able to induce immunological memory responses (World Health Organisation. 1999. *Standardization and validation of serological assays for the evaluation of immune responses to Neisseria meningitidis serogroup AlC vaccines.* Vaccine Development Team of the Department of Vaccine and Biologicals. Ginebra, www.who.int/gpv-documents/; Campagne G. et al. 2000. Safety and immunogenicity of three doses of a Neisseria meningitidis A + C diphtheria conjugate vaccine in infants from Niger. Pediatr. Infect. Dis. J. 19: 144-150; Borrow R. et al. 2000. Induction of immunological memory in UK infants by a meningococcal AlC conjugate vaccine. Epidemiol. Infect. 124: 427- 432; Choo S. et al. 2000. Immunogenicity and reactogenicity of a group C meningococcal conjugate vaccine compared with a group A+C meningococcal polysaccharide vaccine in adolescents in a randomised observer-blind controlled trial. Vaccine 18: 2686- 2692).

The poor immunogenicity of serogroup B capsular polysaccharide has precluded the formulation of vaccines based this compound (Colino J. and Outschoorn I. 1998. Dynamics of the murine humoral immune response to Neisseria meningitidis group B capsular polysaccharide. Infect. Immun. 66: 505-513). The meningococcal serogroup B capsular polysaccharide is a linear homopolymer of sialyc acid consisting of approximately 200 repeated units of α(2-8)-linked N-acetyl neuraminic acid (Bhattacharjee A. K. et al. 1975. Structural determination of the sialic acid polysaccharide antigens of Neisseria meningitidis serogroups B and C with carbon 13 nuclear magnetic resonance. J. Biol. Chem. 250: 1926-1932; Frosch M. and Edwards U. 1993. Molecular mechanism of capsule expression in Neisseria meningitidis serogroup B. p 49-57 in J. Roth, U. Rutishauser and F. A. Troy (ed), Polysialic acid, from microbes to man. Birkhauser Verlag, Basel, Switzerland). These long carbohydrate chains adopt a spatial disposition in an helix form on the surface of the bacterium, adopting a characteristic three-dimensional structure, in whose formation also contribute the proteins and other components of the outer membrane of the bacterium (Colino J. and Outschoorn I. 1998. Dynamics of the murine humoral immune response to Neisseria meningitis group B capsular polysaccharide. Infect Immun 66: 505-513). This homopolymer is not restricted to *N. meningitidis* serogroup B, since is also present in the capsule of *Escherichia coli* K1, a pathogen that causes meningitis in newborn children, in *Pasteurella haemolytica* A2, an important veterinary pathogen and also in *Moraxella non-liquefaciens,* a non-pathogenic microorganism common in nasal graves (Kasper D. L. et al. 1973. Immunochemical similarity between polysaccharide antigens of Escherichia coli 07: K1 (L):NM and group B Neisseria meningitidis. J. Immunol. 110: 262-268; Adlam C. et al. 1986. Purification, characterization and immunological properties of the serotype-specific capsular polysaccharide of Pasteurella haemolytica serotype A7 organisms. J. Gen. Microbiol. 132:1079-1087; Devi S. J. et al. 1991. Identity between polysaccharide antigens of Moraxella non-liquefaciens, group B Neisseria meningitidis and Escherichia coli K1 (non-O acetylated). Infect. Immun. 59: 732-736).

Residues of sialyc acid are also found over the surface of some tissues of the human host. Homopolymers of repeating units of α(2-8)-linked N-acetyl neuraminic acid constitute the only glycosilation of the mammalian neural cell adhesion molecule (NCAM), but in comparison with the meningococcal serogroup B capsular polysaccharide, the carbohydrate chains over the human cells are shorter, being only of approximately 10-50 repeated units (Chuong C. M. et al. 1984. Alterations in neural cell adhesion molecules during development of different regions of the nervous system. J. Neurosci. 4: 2354-2368). Cells with high content of sialyc acid are present in embryos and brains of newborn children, but the content of sialyc acid decreases with age. As a result, the presence of highly sialylated tissues in adults is only limited to isolated regions of some tissues. In certain pathologies, e.g. in aggressive tumors of neuro-ectodermic origin, syalic acid is over-expressed on cells surface (Romero J. D. and Outschoorn I. M. 1997. The immune response to the capsular polysaccharide of Neisseria meningitidis group B. Zentralbl Bakteriol 285: 331-340; Colino J. and Outschoorn I. 1998. Dynamics of the murine humoral immune response to Neisseria meningitis group B capsular polysaccharide. Infect Immun 66: 505-513). *In vitro* experiments indicate that antibodies directed against the capsule of meningococcal serogroup B react with the polysialic acid of human tissues (Finne J. et al. 1987. An IgG monoclonal antibody to group B meningococci cross-reacts with developmentally regulated polysialic acid units of glycoproteins in neural and extraneural tisues. J. Immunol, 138: 4402-4407). This cross-reactivity could explain the low immunogenicity of the meningococcal serogroup B capsular polysaccharide, by a possible development of immunologic tolerance, due to its homology with the polysaccharide present in host tissues. It has been warned of the possibility that anti-meningococcal polysaccharide B antibodies, which *in vitro* have shown recognition of human tissues, potentially would lead to the development of autoimmune diseases. Nevertheless, up to now it has not been possible to demonstrate *in vivo* the effective binding of anti-polysaccharide B antibodies to human tissues, neither has been possible to associate any pathology with the presence of high levels of anti-meningococcal polysaccharide B antibodies circulating in human sera. Several investigations report that, normally, in sera of individuals enjoying good health circulate antibodies recognizing meningococcal polysaccharide B, including mothers who have had healthful children. Besides, no side-effects were reported after the immunization of more than 500 adults and 2000 children with a protein-meningococcal polysaccharide B conjugate vaccine in a study in South-Africa (Mandrell R. E. and Zollinger W. D. 1982. Measurement of antibodies to meningococcal group B polysaccharide: low avidity binding and equilibrium binding constants. J. Immunol 129: 2172-2178; Zollinger W. D. et al. 1984. Safety of vaccines containing meningococcal group B polysaccharide. Lancet 2: 166; Devi S. J. et al. 1991. Antibodies to poly [(2----8)-alpha-N-acetylneuraminic acid] and poly[(2----9)-alpha-N-acetylneuraminic acid] are elicited by immunization of mice with Escherichia coli K92 conjugates: potential vaccines for groups B and C meningococci and E. coli K1. Proc. Natl. Acad. Sci. U S A 88: 7175-7179; Kabat E. A. et al. 1988. The epitope associated with the binding of the capsular polysaccharide of the group B meningococcus and of Escherichia coli K1 to a human monoclonal macroglobulin, IgMNOV. J. Exp. Med. 168: 699-711; Granoff D. M. et al. 1995. Antibody responses to the capsular polysaccharide of Neisseria meningitidis serogroup B in patients with meningococcal disease. Clin. Diagn. Lab. Immunol. 2: 574-582; Romero J. D. and Outschoorn I. M. 1997. The immune response to the capsular polysaccharide of Neisseria meningitidis group B. Zentralbl. Bakteriol. 285: 331-340; Stein D. M. et al. 2005. Are antibodies to the capsular polysaccharide of Neisseria meningitidis group B and Escherichia coli K1 associated with immunopathology?. Vaccine. Article in press).

There are two main reasons for continuing the studies of the use of *N*. *meningitidis* serogroup B capsular polysaccharide as a target for the immune response in a vaccine formulation. Firstly, this is the most conserved structure within the serogroup B, therefore a polysaccharide-based vaccine would protect against infection in a high number of cases, much greater than the number of cases that could be covered by vaccines based on the bacterium outer membrane proteins which induce antibodies of limited cross-reactivity with other strains. Secondly, it is the absence of immunopathological side-effects in individuals with anti-meningococcal polysaccharide B antibodies circulating in sera, as discussed previously.

Several strategies have been used with the objective to increase the immunogenicity of the polysaccharide B (Bartoloni A. et al. 1995. Immunogenicity of meningococcal B polysaccharide conjugated to tetanus toxoid or CRM197 via adipic acid dihydrazide. Vaccine, 13: 463- 470; Devi S. J. et al. 1997. Preclinical evaluation of group B Neisseria meningitidis and Escherichia coli K92 capsular polysaccharide-protein conjugate vaccines in juvenile rhesus monkeys. Infect. Immun. 65: 1045-1052). For example, the immunization with glycoconjugates of a modified serogroup B capsular polysaccharide in which the N-acetyl groups were replaced by N-propionyl groups induced the production of two populations of antibodies: one minority that could be adsorbed with solutions of polysaccharide B purified from bacteria and lacked bactericidal and protective activity. The major population of induced antibodies could not be adsorbed with purified meningococcal polysaccharide B, belonging to the IgG isotype, showed bactericidal activity against several strains from *N. meningitidis* serogroup B, was able to reduce or even eliminate the bacteremia in mice challenged with lethal doses of meningococcus and did not recognize human tissues (Jennings H. J. et a/. 1987. N-propionylated group B meningococcal polysaccharide mimics a unique epitope on group B Neisseria meningitidis. J Exp Med 165: 1207- 1211; Jennings H. J. et al. 1986. Induction of meningococcal group B polysaccharide-specific IgG antibodies in mice by using an N-propionylated B polysaccharide-tetanus toxoid conjugate vaccine. J Immunol 137: 1708-1713; Ashton F. E. et al. 1989. Protective efficacy of mouse serum to the N-propionyl derivative of meningococcal group B polysaccharide. Microb Pathog 6: 455-458; Michon F. et al. 1987. Conformational differences between linear alpha (2----8)-linked homosialooligosaccharides and the epitope of the group B meningococcal polysaccharide. Biochemistry 26: 8399- 8405; Pon R. A. et al. 1997. N-Propionylated group B meningococcal polysaccharide mimics a unique bactericidal capsular epitope in-group B Neisseria meningitidis. J. Exp. Med. 185: 1929-1938). These results indicate the presence of two main epitopes in the polysaccharide B from *N*. *meningitidis:* the first detectable when the polysaccharide is in the form of short chains, as appear in the surface of the human cells, or at random conformation, as is common to find it in the solutions of purified polysaccharide (Diaz R. J. and Outschoorn I. M. 1994. Current status of meningococcal group B vaccine candidates: capsular or noncapsular? Clin. Microbiol. Rev. 7: 559-575). This epitope generate auto-antibodies that are not bactericidal. The second epitope is only detectable when the native. polysaccharide conserves its high molecular mass and is spatially disposed in an helix form, as in the surface of the bacterium. This second epitope, of conformational nature, is responsible for the production of bactericidal and protective antibodies that do no recognize human tissues. It has been reported the isolation of monoclonal antibodies that has been grouped according to their specificity and properties in two populations that correspond exactly with the two previously described (Pon R. A. et al. 1997. N-Propionylated group B meningococcal polysaccharide mimics a unique bactericidal capsular epitope in-group B Neisseria meningitidis. J. Exp. Med. 185: 1929-1938; Granoff D. M. et al. 1998. Bactericidal monoclonal antibodies that define unique meningococcal B polysaccharide epitopes that do not cross-react with human polysialic acid. J Immunol 160: 5028- 5036).

In the solutions of polysaccharide B purified from the bacterium is mainly found the non-conformational, shared with the human N-CAM epitope, which explains explain the low immunogenicity of the polysaccharide B and the little bactericidal activity detected in the antibodies it induces following immunization. The conformational changes introduced in the polysaccharide by the substitution of N-propionyl groups for N-acetyl groups induced the production of a major population of non-autoreactive, bactericidal and protective antibodies. Nevertheless, N-propionylated polysaccharide B, also induces, although in low proportion, a fraction of auto-antibodies, which causes caution in its use as a vaccine, even when a recent study showed the security of its use in humans as immunogen (Bruge J. et al. 2004. Clinical evaluation of a group B meningococcal N-propionylated polysaccharide conjugate vaccine in adult, male volunteers. Vaccine. 22: 1087-1096).

For design of vaccines against meningococcal serogroup B, an alternative to the use of capsule carbohydrates as the target for the immune response is the use as immunogens of peptide sequences that constitute molecular mimetics (mimotopes) specific for the conformational epitope present in polysaccharide B, responsible of protection and present when the carbohydrate is in its native conformation on the surface of the bacterium.

An approach to identify mimotopes of capsular polysaccharides is panning with antibodies random-peptide libraries displayed in filamentous phages. This technology is based on the capacity of filamentous phages to expose in their surface foreign peptide sequences fused to proteins of the viral capsule. The peptide sequences displayed in the surface of the viral particles are easily accessible and potentially able to bind to the molecules used for selection, and therefore they can be selected on the basis of this affinity. The sequence of a peptide from a phage-library selected by a certain property can easily be deduced from the nucleotide sequence of the genome of the selected phage (Parmley S. F. et al. 1988. Antibody-selectable filamentous fd phage vectors: affinity purification of target genes. Gene 73:305-318; Smith G. P. et al. 1993. Libraries of peptides and proteins displayed on filamentous phage. Methods Enzymol. 217: 228-257; Cwirla S. E. et al. 1990. Peptides on phage: a vast library of peptides for identifying ligands. Proc. Natl. Acad. Sci. U S A; 87: 6378-6382). To be considered as possible vaccine candidates these peptides must be able, in first place, to bind specific antibodies directed against the original antigen and to inhibit the binding of these antibodies to the antigen. Secondly, they must induce an immune response characterized by the presence of antibodies that recognize the pathogen and protect against the infections it causes. These peptides have defined and reproducible structures and can replace the natural antigen in a vaccine elaboration, with the additional advantage that the target of the antibodies they induce is well defined, the possibility of induction of auto-antibodies able to recognize human tissues, is eliminated, thus evading the possibility of development of autoimmune diseases (Monzavi-Karbassi B. et al. 2002. Peptide mimotopes as surrogate antigens of carbohydrates in vaccine discovery. Trends. Biotechnol. 20:207-214), This would be particularly important in the case of the capsular polysaccharide from serogroup B of *N. meningitidis.*

On the other hand, it is possible the direct immunization of experimental animals with phage-exposed peptides. This accelerates the process of immunological evaluation of possible vaccine candidates, since is possible to avoid the traditional procedure of peptide synthesis and conjugation to protein carriers. It has been demonstrated that the filamentous phages are potent carrier proteins, able to present effectively peptides to the immune system (Meola A. et al. 1995. Derivation of vaccines from mimotopes: Immunologic properties of human Hepatitis B virus surface antigen mimotopes displayed on filamentous phage. J. Immunol. 154: 3162-3172: de la Cruz V. F. et al. 1988. Immunogenicity and epitope mapping of foreign sequences via genetically engineered filamentous phage. J. Biol. Chem. 263: 4318-4322; Greenwood J. et al. 1991. Multiple display of foreign peptides on a filamentous bacteriophage. Peptides from Plasmodium falciparum circumsporozoite protein as antigens. J. Mol. Biol. 220: 821-827: Willis A. E. et al. 1993. Immunological properties of foreign peptides in multiple display on a filamentous bacteriophage. Gene 128: 79-83; Galfrè G. et al. 1996. Immunization with phage-displayed mimotopes. Methods Enzymol. 267: 109-115; Bastien N. et al. 1997. Protective immune responses induced by the immunization of mice with a recombinant bacteriophage displaying an epitope of the human respiratory syncytial virus. Virology 234:118-122; Menéndez T. et al. 2001. Immunisation with phage-displayed variable region 2 from meningococcal PorA outer membrane protein induces bactericidal antibodies against Neisseria meningitidis. Immunol. Lett. 39: 143-148).

Using this methodology, several peptides that constitutes molecular mimetics of capsular polysaccharides from *Cryptococcus neoformans, Streptococcus* group A, *Streptococcus* group B and the lipooligosaccharide from *Shigella flexneri,* among others, has been identified (Valadon P. et al. 1996. Peptide libraries define the fine specificity of anti-polysaccharide antibodies to Cryptococcus neoformans. J. Mol. Biol. 261: 11-22; Harris S. L. et al. 1997. Exploring the basis of peptide-carbohydrate crossreactivity: evidence for discrimination by peptides between closely related anti-carbohydrate antibodies. Proc. Natl. Acad. Sci. USA 94: 2454-2459; Pincus S. H. et al. 1998. Peptides that mimic the group B Streptococcal type III capsular polysaccharide antigen. J. Immunol. 160: 293-298; Phalipon A. et al. 1997. Induction of anti-carbohydrate antibodies by phage library-selected peptide mimics. Eur. J. Immunol. 27: 2620-2625).

In the field of vaccine development against *N*. *meningitidi,* there also has been work concerning the identification of molecular mimetics of the capsular polysaccharides of bacteria. For example, it was reported the identification of molecular mimetics of the capsular polysaccharide from serogroup A, selected from a random peptide library displayed in filamentous phages, with a monoclonal antibody specific for this polysaccharide. The mimotopes were able to inhibit the binding of human hyperimmune sera to the natural antigen and to induce the production of high levels of anti-polysaccharide antibodies after the immunization of experimental animals (Grothaus M. C. et al. 2000. Selection of an immunogenic peptide mimic of the capsular polysaccharide of N. meningitidis serogroup A using a peptide display library. Vaccine 18: 1253-1263). In the case of meningococcal serogroup C, the synthesis of a peptide with a sequence corresponding to the hypervariable regions of light and heavy chains of an anti-idiotype antibody, mimetic of meningococcal polysaccharide C and immunization with said synthetic peptide, resulted in high levels of protective anti-meningococcal polysaccharide C antibodies detected in the sera of immunized animals (Westerink M. A. et al. 1995. Peptide mimicry of the meningococcal group C capsular polysaccharide. Proc. Natl. Acad. Sci. U S A 92: 4021-4025).

The application of the technology of identification of mimotopes is considered of vital importance in the search for a vaccine candidate against the serogroup B of *N. meningitidis,* due to the possibilities to direct the immune response exactly against the conformational and protector epitope present in the polysaccharide of said serogroup, that is not shared with the human N-CAM. It was reported the identification of such structures using four monoclonal antibodies and two libraries of linear and cyclic seven- aminoacids-long peptides, but the immunological evaluation of such structures was not reflected in the publication (Shin J. S. et al. 2001. Monoclonal antibodies specific for Neisseria meningitidis group B polysaccharide and their peptide mimotopes. Infect. Immun. 69: 3335-3342). Later, in a publication of the year 2004, using one of the monoclonal antibodies of the former work and a phage-library displaying linear 12 aminoacids-long peptides the identification of three new structures was reported and the immunological evaluation was carried out in animal models. Sera recognized very well the serogroup B meningococcus, but the evaluation of the functional activity of these antibodies was not reflected in the publication (Park I. et al. 2004. Peptide mimotopes of Neisseria meningitidis serogroup B capsular polysaccharide. Yonsei Medical Journal. 45: 755-758). Granoff and Moe (Granoff D. M. and Moe G. R. 2003. Molecular mimetics of unique Neisseria meningitidis serogroup B epitopes. US Pat. 6,642,354 B2), protected the use in vaccine formulations of 67 peptide sequences, that were selected from peptide libraries displayed in phages, on the basis of their capacity to bind monoclonal antibodies directed to serogroup B capsular polysaccharide. Two of them, selected for immunological studies, were synthesized and conjugated to outer membrane vesicles from *N. meningitidis,* purified from a meningococcal serogroup B strain mutant of capsule. It was informed in this patent that immunization with one of this conjugates induced the production of antibodies that showed bactericidal activity against *N. meningitidis* of serogroup B meningococci. Taking into account that this peptide was conjugated to outer membrane vesicles from *N. meningitidis,* whose main components are the proteins from the outer membrane of the bacterium, and that it is well documented their capacity of induction of antibodies with bactericidal activity against meningococci, it is difficult the interpretation of these results. The use of OMV as carrier in this study hampers the direct evaluation of the capacity of this peptide to induce antibodies with bactericidal activity. The authors said that a fraction of the bactericidal activity detected in these sera is due to the presence of anti-peptide antibodies, since incubation of said sera with the unconjugated peptide increased the bacterial survivors in the assay. Nevertheless, in the scientific publications that reflect these results they informed that the antibodies elicited after immunization with the conjugates peptide-OMV recognized weakly the original antigen and the evaluation of bactericidal activity of sera was not reported (Moe G. R. et al. 1999,Molecular mime tics of polysaccharide epitopes as vaccine candidates for prevention of Neisseria meningitidis serogroup B disease. FEMS Immunol. Med. Microbiol. 26: 209-269;.Moe G. R and Granoff D. M. 2001. Molecular mimetics of Neisseria meningitidis serogroup B polysaccharide. Int. Rev Immunol. 20:201-20).

The alternative of the identification of mimotopes, specifically in the field of vaccines against *N. meningitidis* of serogroup B, is important since the peptides selected according to this methodology, even when being completely different with respect to the original antigen, are able to lead to the production of antibodies against it in their natural context, that is, on the surface of the bacterium, where the conformational and protective epitope of B polysaccharide is formed. These peptides induce the production of antibodies with bactericidal and protective activity against *N. meningitidis.* Moreover, since the antibodies that elicited these peptides do not cross-react with the epitope formed by short chains of poly-sialic acid present on certain human tissues, the possibility to evoke an autoimmune response is eliminated. They constitute, therefore, an alternative source of antigens allowing the development of a new generation of reagents with potential use in the diagnosis, the therapy or the prevention of diseases.

### DETAILED DESCRIPTION OF THE INVENTION

In the present invention the peptides NMGBPS1, NMGBPS2, NMGBPS3 and NMGBPS4 are reported for the first time as components of reagents for the diagnosis or for a vaccine formulation, of therapeutic or preventive character against the meningococcal disease or any infection caused by a member of the genus *Neisseria* or by any other bacteria, viruses or parasites.

The novelty of this invention resides in the description for the first time of peptides NMGBPS1, NMGBPS2, NMGBPS3, and NMGBPS4 as molecular mimotopes of epitopes present in the capsular polysaccharide of *Neisseria meningitidis* serogroup B, able to induce the production of bactericidal antibodies against meningococcus strains of this serogroup. Said peptides can be used as reagents for the diagnosis and/or in vaccine formulations to treat or to prevent the disease caused by members of the genus Neisseria or other bacteria, viruses or parasites. In the present invention the aminoacids are referred to using the one-letter code (e.g. A-Alanine, W-Tryptophan, S-Serine, E- Glutamic Acid, Y-Tyrosine, K-Lysine, F-Phenylalanine, I-Isoleucine, V-Valine, P-Proline, etc).

### BRIEF DESCRIPTION OF THE DRAWINGS

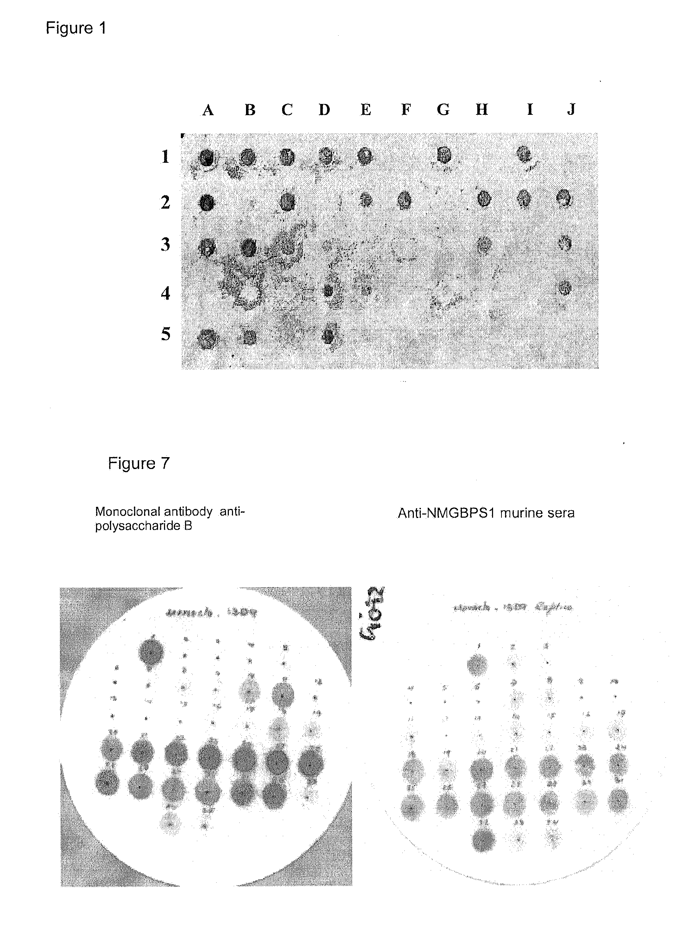

Figure 1. The phages selected after panning of phage-displayed peptides library, with the monoclonal antibody anti-polysaccharide B, were applied onto a nitrocellulose membrane and allowed to react with the selector antibody. Each point corresponds to 30 µg of a selected individual phage clone. The control phage was applied in positions G5 and I5.

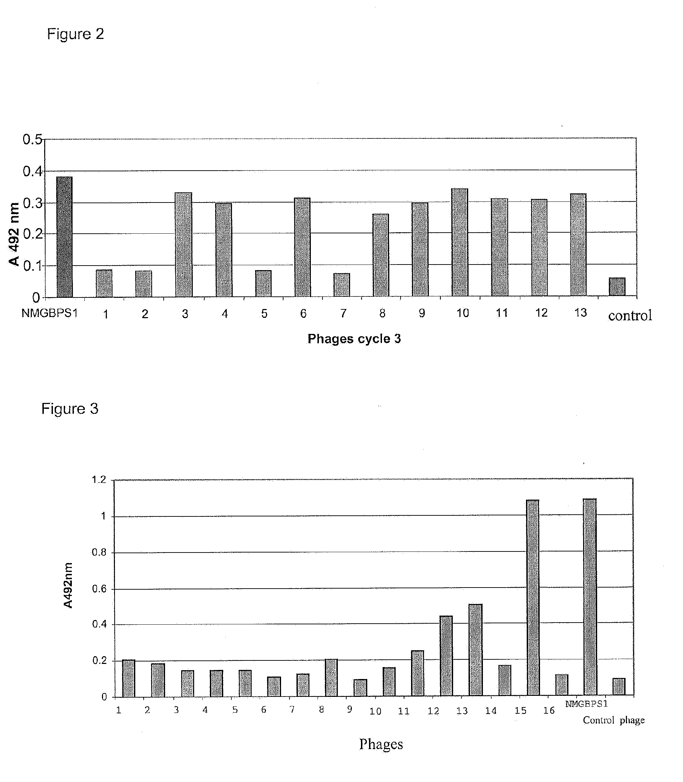

Figure 2. Evaluation of ELISA reactivity of phages adsorbed to the selector antibody after three rounds of panning of the library. Phages were purified individually and used to coat the ELISA plates. The graphic shows the ELISA absorbance at 492 nm (A₄₉₂ₙₘ) for 13 of the evaluated phages.
Figure 3. Evaluation of ELISA reactivity of phages adsorbed to the selector antibody after two rounds of panning of library. Phages were purified individually and used to coat the ELISA plates. Graphic shows the ELISA absorbance at 492 nm (A₄₉₂ₙₘ) for 16 of the evaluated phages.

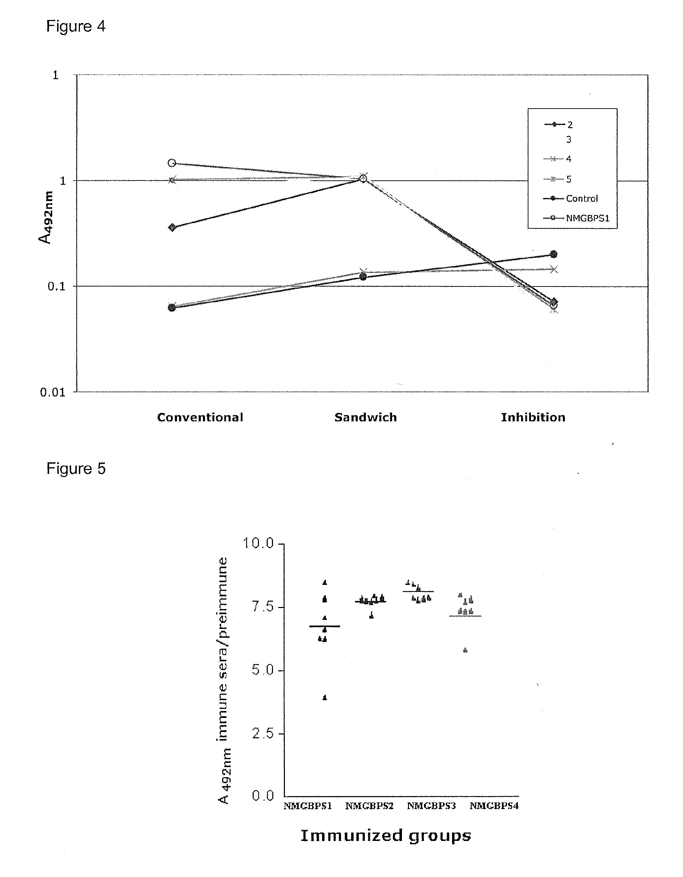

Figure 4. Evaluation of reactivity, using three ELISA system: conventional, sandwich and inhibition, according to explanation in the Example 9, of phages which display in their surfaces the peptides selected by panning the phage-displayed peptide library with the monoclonal antibody anti-polysaccharide B
Figure 5. Levels of IgG antibodies induced after immunization of Balb/c mice with peptides NMGBPS1-NMGBPS4, displayed in filamentous phages, evaluated by ELISA. The graphic shows the increases of specific antibodies detected in the immune sera, collected after three doses with respect to the corresponding preimmune sera, collected before immunizations, for groups immunized with each one of the phages.

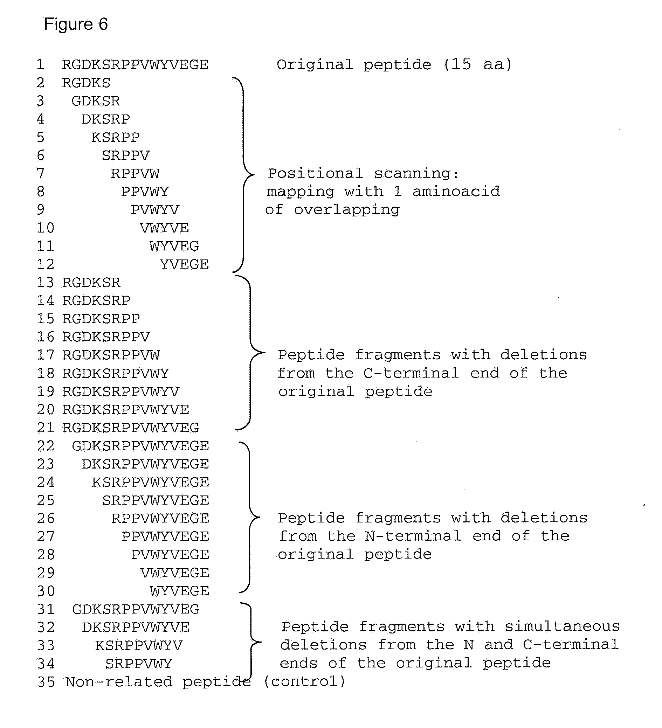

Figure 6. Peptide fragments synthesized on cellulose membranes for the determination of the epitopes recognized by the monoclonal antibody anti-polysaccharide B and the murine sera induced against the peptide NMGBPS1.
Figure 7. Reactivity of the monoclonal antibody anti-polysaccharide B (left panel) and murine sera induced against the peptide NMGBPS1 (right panel) with a collection of peptide fragments derived from peptide NMGBPS1. The design of the collection of peptide fragments is shown in Figure 6.

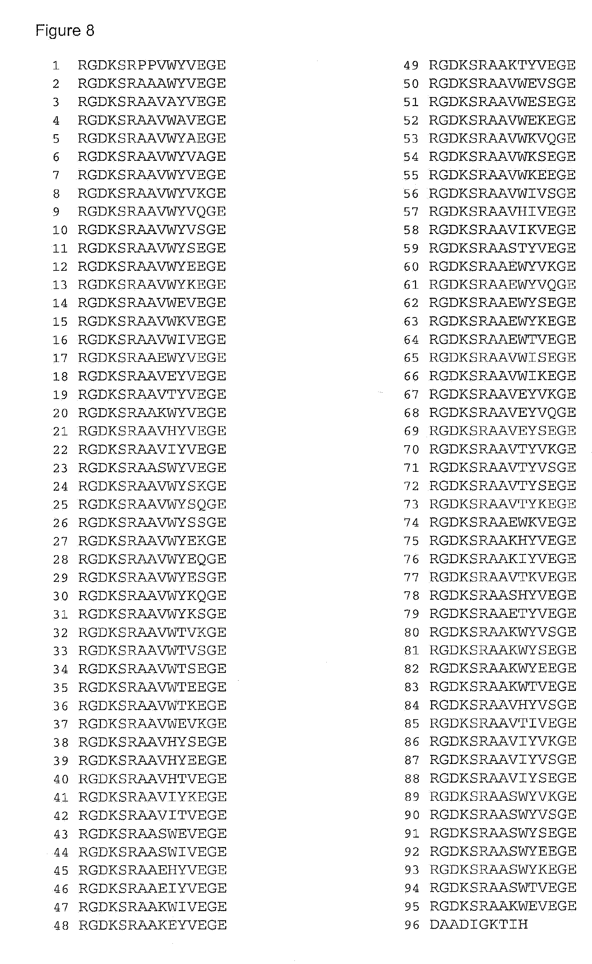

Figure 8. Collection of peptides synthesized on pins, derived from modifications introduced to the peptide NMGBPS1 consisting of: a) the simultaneous substitution of the Pro in positions 7 and 8 by an Ala in each position, and b) the introduction of 1 to 3 point substitutions in the sequence binding the monoclonal antibody anti-polysaccharide B. Peptide 1 corresponds with the sequence of NMGBPS1.

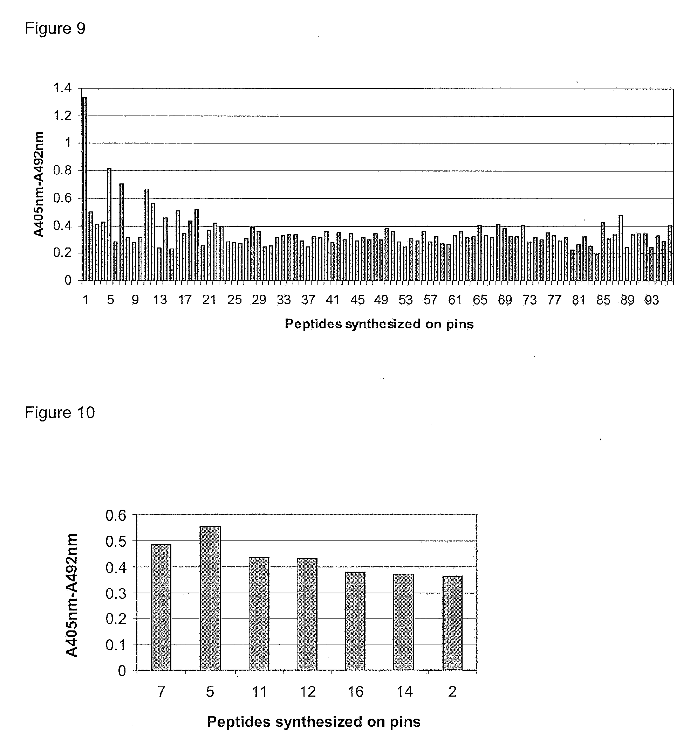

Figure 9. Reactivity of the collection of peptides synthesized on pins showed in Figure 8, with the monoclonal antibody anti-polysaccharide B. The identification numbers of the peptides are the same as in the list showed in Figure 8. Bar 1 shows reactivity with peptide NMGBPS1.
Figure 10. Reactivity with the monoclonal antibody anti-polysaccharide B of peptides derived from the sequence of NMGBPS1. Substitutions for each peptide were as follows: V₁₂ by A (peptide 5), V₁₂ by S (peptide 11), V₁₂ by E (peptide 12), V₉ by A (peptide 16), Y₁₁ by E (peptide 14), Y₁₁ by I (peptide 2). In every case P₇ and P₈ were simultaneously substituted by A at each position. The identification numbers of the peptides are the same as in the list showed in Figure 8. Bar 1 (peptide 7) shows the reactivity of peptide NMGBPS1 with P₇ and P₈ simultaneously substituted by A at each position.

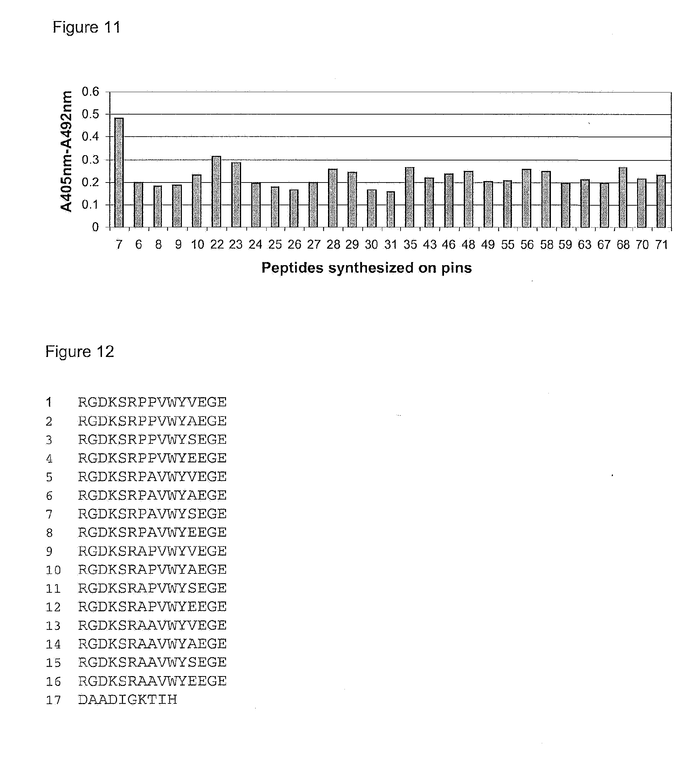

Figure 11. Reactivity of the anti-polysaccharide B monoclonal antibody with peptides based on the sequence NMGBPS1, where the residue E₁₃ and additional residues were replaced in Figure 8. In all the cases P₇ and P₈ were simultaneously replaced by A in each position. The numbers of peptides correspond with the list shown in Figure 8. Bar 1 (peptide 7) shows the reactivity of peptide NMGBPS1 with P₇ and P₈ simultaneously substituted by A at each position
Figure 12. Peptide collection synthesized on pins, derived from modifications introduced to peptide NMGBPS1 consisting of a) the substitution of V₁₂ by A₁₂, S₁₂ and E₁₂, and b) the substitution of P₇ by A₇ or P₈ by A₈. Peptide 1 corresponds with the sequence of NMGBPS1.

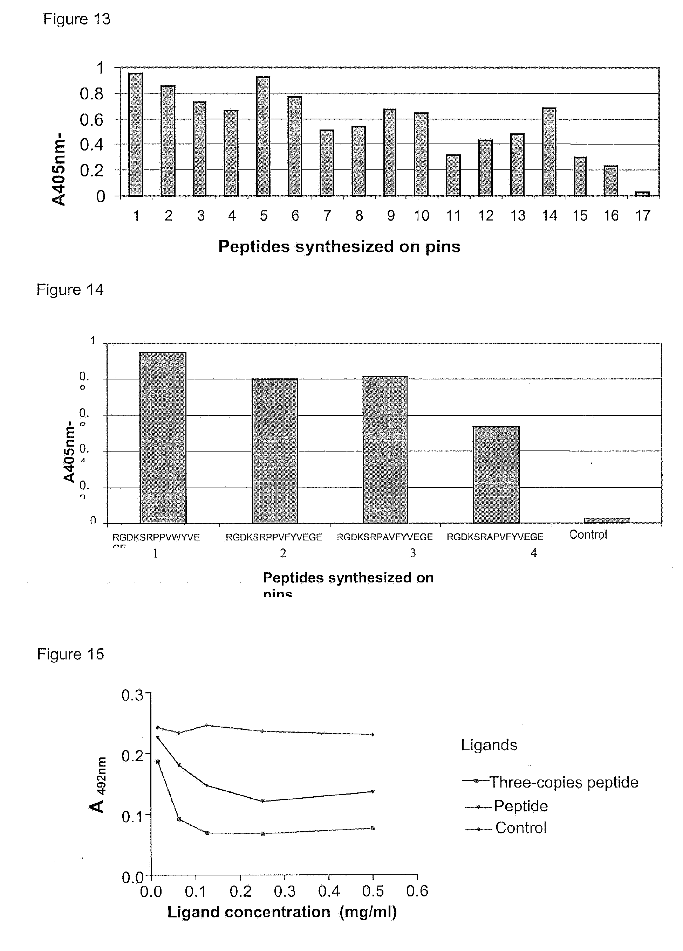

Figure 13. Reactivity of the peptide collection synthesized on pins, showed in Figure 12, with the monoclonal antibody anti-polysaccharide B.
Figure 14. Reactivity of the anti-polysaccharide B monoclonal antibody with peptides based on the sequence NMGBPS1 (bar 1), in which the following modifications were introduced 1) the substitution of W₁₀ by F₁₀, and 2) the substitution of P₇ by A₇ or P₈ by A₈. The graphic shows also the sequence of each peptide.
Figure 15. Inhibition of binding of the anti-polysaccharide B monoclonal antibody to the capsular polysaccharide of *Neisseria meningitidis* serogroup B, using as ligands a) a peptide containing 3 copies in tandem of the minimal sequence necessary for the binding of the peptide NMGBPS1 to the anti-polysaccharide B monoclonal antibody, b) the peptide NMGBPS1 and c) a control peptide

### DETAILED DESCRIPTION OF THE PREFERRED EMBODIMENTS OF THE INVENTION

### Example 1 Panning of the peptide library displayed on phages with a monoclonal antibody anti-polysaccharide B of Neisseria meningitidis.

For the selection of peptides described in the present invention, a linear random peptide library of 15 aminoacid residues, expressed in the P8 region of filamentous phages, was constructed. Said library was panned using as selective molecule a murine monoclonal antibody with bactericidal activity against *N*. *meningitidis* serogroup B that does not recognize human tissues (Pon R. A. et al. 1997. N-Propionylated group B meningococcal polysaccharide mimics a unique bactericidal capsular epitope in-group B Neisseria meningitidis. J. Exp. Med. 185: 1929-1938).

The panning of the library was carried out in cycles essentially as described in the literature (Smith G. P. et al. 1993. Libraries of peptides and proteins displayed on filamentous phage. Methods Enzymol. 217: 228-257), with the exception that the antibody was directly used to coat the solid support.

After the reaction of phages and the monoclonal antibody, the phage fraction not adsorbed to the antibody (fraction N1) was collected and tittered. Also the phage fraction adsorbed to the antibody (fraction A1) was collected and the titer of phages was determined. The ratio titer N1/ titerA1 was calculated. The fraction A1 was amplified and allowed to react with the anti-polysaccharide B monoclonal antibody and the phage fractions adsorbed and not adsorbed to the antibody were collected and tittered again. These cycles were repeated until the ratio titer N/ titer A was less than 10³. Four cycles of panning were made. Table 1 shows the values obtained after titration of fractions A and N collected after each cycle of panning. The table also shows the titter of fraction A obtained in each cycle of panning after being amplified and purified to be used in the following cycle.

**Table 1. Titers, expressed as Transduction Units of ampicillin resistance (TU/mL) of fractions A and N, obtained after each cycle of panning and the corresponding titer ratios N/A. Aₐₘₚ: amplified and purified fraction A.**

| Cycle | N | A | N/A | Aₐₘₚ |
|---|---|---|---|---|
| 1 | 10¹¹ | 10² | 10⁹ | 7X10⁹ |
| 2 | 10⁸ | 10⁴ | 10⁴ | 10¹⁰ |
| 3 | 10⁹ | 10⁶ | 10³ | 10⁸ |
| 4 | 7X10⁶ | 2X10⁵ | 35 | |

Phages adsorbed to the selector antibody after four library panning cycles, were used to infect *E*. *coli* XL-1 blue cells and several dilutions were spilled on agar plates containing the culture media 2XYT. Individual phages were selected and amplified in liquid cultivations. The phages were purified by precipitation with polyethylene glycol.

### Example 2. Reactivity of the selector antibody with phages selected after four library panning cycles.

Purified phages (30 µg) were applied onto a nitrocellulose membrane. An equal amount of control phage was also applied. After blocking with skimmed milk, the membrane, was incubated with the anti-polysaccharide B monoclonal antibody. The reactivity was detected, by incubation with antibodies specifically recognizing mice IgG conjugated to the enzyme horseradish peroxidase, with a solution containing H₂O₂ and the chromogen diaminobencidine. Figure 1 shows the results. Twenty-five phages, corresponding to the points of greater intensity were selected for further work.

### Example 3. Purification and sequencing of DNA from phages selected after four library panning cycles with the anti-polysaccharide B monoclonal antibody.

The DNA of 25 phages positive in the immunoidentification experiment was purified and the nucleotide sequence was determined. The purification of phage DNA was carried out as previously described (Sambrook J. et al. 1989. Molecular Cloning, a Laboratory Manual, 2nd edn, Cold Spring Harbor Laboratory Press, Cold Spring Harbor, New York, USA). Viral DNA was sequenced with the automatic sequencer ALFexpressll (Termo Sequenase^{™} Cy^{™} 5 Dye Terminador Kit, Amersham Biosciences), using the oligonucleotides M13/pUC Sequencing primer (-47) #1224, New England Biolabs Inc., USA) and M13/pUC Reverse sequencing primer (-48) #1233, New England Biolabs Inc., USA). All the clones had the same sequence, as shown in the sequence list (Sequence identification No 1). The peptide corresponding to the translation of this DNA sequence is shown in the sequence list (Sequence identification No 5).

### Example 4. Characterization of the peptide sequence displayed in the phages selected after four library panning cycles with the anti-polysaccharide B monoclonal antibody.

For the analysis of the sequence displayed in phages selected after four library panning cycles, an alignment was made with other sequences of molecular mimetics of the capsular polysaccharide from *N. meningitidis* serogroup B previously reported (Shin J. S. et al. 2001. Monoclonal antibodies specific for Neisseria meningitis group B polysaccharide and their peptide mimotopes. Infect. Immun. 69: 3335-3342; Park I. et al. 2004. Peptide mimotopes of Neisseria meningitidis serogroup B capsular polysaccharide. Yonsei Medical Journal. 45: 755-758; Moe G. R. et al. 1999. Molecular mimetics of polysaccharide epitopes as vaccine candidates for prevention of Neisseria meningitidis serogroup B disease. FEMS Immunol. Med. Microbiol. 26: 209-269; Moe G. R and Granoff D. M. 2001. Molecular mimetics of Neisseria meningitidis serogroup B polysaccharide. Int. Rev .Immunol. 20:201-20; Granoff D. M. and Moe G. R. 2003. Molecular mimetics of unique Neisseria meningitidis serogroup B epitopes. USP 6,642,354 B2), not being in any case the percents of similarity greater than 29%. The alignment was performed with the software Clustal W (Thompson J. D. et al. 1994. CLUSTAL W: improving the sensitivity of progressive multiple sequence alignment through sequence weighting, position-specific gap penalties and weight matrix choice. Nucleic Acids Res. 22:4673-80).

The sequence was also characterized by similarity searches in the databases of NCBI using the software BLASTP 2.2.10 (Altschul S. F. et al. 1997. Gapped BLAST and PSI-BLAST: a new generation of protein database search programs. Nucleic Acids Res. 25:3389-3402). The search was limited to sequences of bacterial genes and proteins contained in the databases SwissProt (http://www.ebi.ac.uk/swissprot/) and NCBI (http://www.ncbi.nlm.nih.gov/). The results of this procedure indicated that percentages of similarity of peptide NMGBPS1 with the sequences deposited in these databases were not greater than 30%.

### Example 5. Reactivity of the selector antibody with phages selected after the third cycle of library panning.

To identify other mimetic structures of the capsular polysaccharide from *N*. *meningitidis* serogroup B, phages adsorbed to the anti-polysaccharide B antibody after the third cycle of library panning were evaluated by ELISA. Phages were purified individually and used to coat ELISA plates. In the experiment were also included the control phage and the phage displaying the peptide NMGBPS1. After blocking with skimmed milk, plates were incubated with the anti-polysaccharide B monoclonal antibody. The reactivity was revealed, after incubating with antibodies specific for mice IgG, conjugated to the enzyme horseradish peroxidase, with a solution that contained H₂O₂ and the chromogen o-phenylene-diamine (OPD). The reaction was stopped with sulfuric acid 2.5 N and the A₄₉₂ₙₘ was read in an ELISA plate reader. Figure 2 shows the results obtained for several of the purified phages. The twenty phages with ELISA signals of greater intensity than that of the control phage, were selected for further work.

### Example 6. Purification and sequencing of DNA from phages selected after the third cycle of library panning with anti-polysaccharide B monoclonal antibody.

The DNA from the 20 selected phages in the immunoidentification ELISA experiment were purified and sequenced, following identical procedure to that described in the Example 3. All the clones had the same sequence as that from phages isolated after the fourth cycle of library panning. This sequence is shown in the list of sequences with the No. of identification of sequence 1. The aminoacid sequence corresponding to the translation of this DNA sequence to protein sequence is shown in the list of sequences with the No. of identification of sequence 5

### Example 7. Reactivity of the selector antibody with phages selected after the second cycle of library panning.

To continue the search for other mimetic structures of the capsular polysaccharide from *N. meningitidis* serogroup B, phages adsorbed to the anti-polysaccharide B antibody after the second cycle of library panning were evaluated by ELISA. Phages were purified individually and used to coat ELISA plates. In the experiment were also included a control phage and a phage displaying the peptide NMGBPS1. After blocking with skimmed milk, plates were incubated with the anti-polysaccharide B monoclonal antibody. Reactivity was detected by incubating with antibodies specific for mice IgG, conjugated to the enzyme horseradish peroxidase, with a solution that contained H₂O₂ and the chromogen o-phenylene-diamine (OPD). The reaction was stopped with sulfuric acid 2.5 N and the A₄₉₂ₙₘ was read in an ELISA plate reader. Figure 3 shows the results obtained for several of the purified phages. Fifteen phages with ELISA signals of greater intensity than that from the control phage, were selected for further work.

### Example 8. Purification and sequencing of DNA from phages selected after the second cycle of library panning with the anti-polysaccharide B monoclonal antibody.

The DNA from the 15 phages selected in the immunoidentification ELISA experiment, were purified and sequenced following an identical procedure as described in Example 3. A total of 5 unique sequences were identified. Table 2 summarizes the frequency of appearance of every sequence displayed in the phages. Sequence 1 corresponds to peptide NMGBPS1.

**Table 2. Five unique sequences displayed in the filamentous phages were identified after two cycles of peptide library panning with the anti-polysaccharide B monoclonal antibody. Sequence 1, identical to that of peptide NMGBPS1, appeared with the highest frequency**

| Unique sequences | Number of identical clones | Phage name | No identification of sequence | |
|---|---|---|---|---|
| | | | DNA | Protein |
| 1 | 7 | NMGBPS1 | 1 | 5 |
| 2 | 1 | NMGBPS2 | 2 | 6 |
| 3 | 1 | NMGBPS3 | 3 | 7 |
| 4 | 1 | | | |
| 5 | 4 | NMGBPS4 | 4 | 8 |

### Example 9. ELISA characterization of reactivity of peptides exposed in phages selected by library panning.

Phages expressing each one of the unique sequences identified in Example 8 were purified and their reactivity with the anti-polysaccharide B monoclonal antibody was studied. Three ELISA experiments were carried out: 1) Conventional ELISA where the phages were applied directly onto the polypropylene plates, further the anti-polysaccharide B monoclonal antibody was added and finally immunoglobulins specific for mice IgG were added; 2) ELISA sandwich where the anti-polysaccharide B monoclonal antibody was applied directly onto ELISA plates, then the phages, later an anti wild-type M13-phage serum obtained in rabbits and finally immunoglobulins specific for rabbit IgG; 3) Inhibition ELISA where the plates were coated with a solution of polysaccharide B purified from the bacterium. Mixtures of the anti-polysaccharide B monoclonal antibody with the phages were added and finally immunoglobulins specific for mice IgG were added. In all cases the immunoglobulins added at the end were conjugated to the enzyme horseradish peroxidase and the reactivity was revealed with a solution containing H₂O₂ and the chromogen o-phenylene-diamine (OPD). The reaction was stopped with sulfuric acid 2.5 N and the A₄₉₂ₙₘ was read in an ELISA plate reader. Figure 4 shows the results obtained. The phages clones expressing the unique sequences 2, 3 and 5, as shown in Table 2, behaved in a way similar to peptide NMGBPS1 in the three ELISAs assayed, whereas the phage expressing the unique sequence 4, showed in Table 2, behaved in a way similar to that of control phage in the three ELISAs assayed. Peptides 2, 3 and 5, selected for further work, were renamed as NMGBPS2, NMGBPS3 and NMGBPS4, respectively.

The DNA sequences corresponding to phages NMGBPS12, NMGBPS3 and NMGBPS4 are shown in the list of sequences with the No. of identification of sequences: 2, 3 and 4, respectively. The peptides corresponding to the translation of these sequences of DNA to protein sequences are shown in the list of sequences with the No. of identification of sequences 6, 7 and 8, respectively. Table 2 shows the correspondence between the identified phages and the identification numbers of sequences.

### Example 10. Characterization of the peptide sequences expressed in the phages selected after the second cycle of library panning with the anti-polysaccharide B monoclonal antibody

For the analysis of the sequences exposed in the phages selected after the second cycle of library panning, an alignment was made with other sequences of mimetic structures of the *N. meningitidis* polysaccharide B, previously reported (Shin J. S. et al. 2001. Monoclonal antibodies specific for Neisseria meningitidis group B polysaccharide and their peptide mimotopes. Infect. Immun. 69: 3335-3342; Park I. et al. 2004. Peptide mimotopes of Neisseria meningitidis serogroup B capsular polysaccharide. Yonsei Medical Journal. 45: 755-758; Moe G. R. et al. 1999. Molecular mimetics of polysaccharide epitopes as vaccine candidates for prevention of Neisseria meningitidis serogroup B disease. FEMS Immunol. Med. Microbiol. 26: 209-269; Moe G. R and Granoff D. M. 2001. Molecular mimetics of Neisseria meningitidis serogroup B polysaccharide. Int. Rev .Immunol. 20:201-20; Granoff D. M. and Moe G. R. 2003. Molecular mimetics of unique Neisseria meningitidis serogroup B epitopes. USP 6,642,354 B2) resulting in any case with similarity percentages greater than 30% for peptide NMGBPS2, neither greater than 33 % for peptide NMGBPS3 and nor greater than 40 % for peptide NMGBPS4. The alignments were done with the Clustal W program (Thompson J. D. et al. 1994. CLUSTAL W: improving the sensitivity of progressive multiple sequence alignment through sequence weighting, position-specific gap penalties and weight matrix choice. Nucleic Acids Res. 22:4673-80).

The sequences obtained were also characterized by similarity search in the database of the NCBI using program BLASTP 2,2,10 (Altschul S. F. et al. 1997. Gapped BLAST and PSI-BLAST: a new generation of protein database search programs. Nucleic Acids Res. 25: 3389-3402). The search was limited to the sequences of bacterial genes and proteins contained in the data bases SwissProt (http://www.ebi.ac.uk/swissprot /) and NCBI (http://www.ncbi:nlm.nih.gov /). The results of this procedure indicated that percentages of similarity detected against the sequences deposited in these databases were less than 30% for peptides NMGBPS 2 and 4, and less than 35% for peptide NMGBPS3.

### Example 11. Immunization of Balb/c mice by intraperitoneal route with the phages displaying in their surfaces the peptides NMGBPS1-NMGBPS4

To evaluate the immunogenicity of peptides NMGBPS1-NMGBPS4, an immunization schedule in Balb/c mice was conducted, where animals were directly inoculated with phages exposing said peptides. The phages were purified by caesium chloride gradient, following a modification of the purification procedure 1 (Lin T.C. et al. 1980. Isolation and characterization of the C and D proteins coded by gene IX and gene VI in the filamentous bacteriophage fl and fd. J. Biol. Chem. 255: 10331-10337) described by de la Cruz and collaborators (de la Cruz F.V. et al. 1988. Immunogenicity and epitope mapping of foreign sequences via genetically engineered filamentous phage. J. Biol. Chem. 25: 4318-4322).

A group of 7 Balb/c mice (H-2^{d} feminine sex, 7-8 weeks of age) was immunized with each phage. Each mouse received 10¹¹ viral particles in each inoculation. An additional group of mice was immunized with 5 ug per mouse in each inoculation of N-propionylated polysaccharide B from *N. meningitidis* (pPSC-B) conjugated to the protein human serum albumin (HSA) (pPSC-B/HSA) (Jennings H. J. et al. 1986. Induction of meningococcal group B polysaccharide-specific IgG antibodies in mice by using an N-propionylated B polysaccharide-tetanus toxoid conjugate vaccine. J Immunol 137: 1708-1713). Three immunizations by intraperitoneal route were made, separated by an interval of 15 days. All the immunogens were emulsified with Complete Freund Adjuvant in the first dose and with Incomplete Freund Adjuvant in the successive doses. Table 3 describes the immunogen received by each group.

**Table 3 Groups of mice and inmunogens used for the immunological evaluation of peptides selected from the panning of the peptide library exposed in phages, with the anti-polysaccharide B monoclonal antibody.**

| Groups | Immunogen |
|---|---|
| 1 | Phage NMGBPS 1 |
| 2 | Phage NMGBPS 2 |
| 3 | Phage NMGBPS 3 |
| 4 | Phage NMGBPS 4 |
| 5 | Phage control |
| 6 | pPSC-B/HSA |

### Example 12. ELISA evaluation of immunogenicity of peptides NMGBPS1-NMGBPS4

The levels of antibodies (IgG) induced by peptides NMGBPS1- NMGBPS4 exposed in filamentous phages were evaluated by ELISA. The sera from the animals, obtained after the third inoculation, were evaluated using synthetic peptides with the sequences of the identified peptides as antigen for coating the ELISA plates. Figure 5 shows that the levels of specific IgG antibodies against the peptides displayed in the phages, after three immunizations increased significantly. The results were analyzed by the non-parametric method of analysis of variance of simple classification by ranks of Kruskal-Wallis, because the variances between the groups were not homogenous according to the Bartlett Test. In order to make non-parametric multiple comparisons the Dunns Test was used.

### Example 13. Evaluation of serum bactericidal activity of the antibodies induced by peptides selected from the phage-displayed peptide library after panning with the anti-polysaccharide B monoclonal antibody.

Mixtures of sera from each group of mice, inoculated with each immunogen, were evaluated by serum bactericidal assay by the assay of Ashton and collaborators (Ashton F. E. et al. 1989. Protective efficacy of mouse serum to the N-propionyl derivative of meningococcal group B polysaccharide. Microb. Pathog. 6: 455-458), for the evaluation of antibodies directed against the meningococcal polysaccharide B, but instead of using the buffer solution recommended by these investigators, Veronal solution supplemented with BSA was used. This solution is recommended to increase the sensitivity of the bactericidal assay when antibodies directed against the capsule of *N. meningitidis* serogrupo B are evaluated (Mandrell R. E. et al. 1995. Complement-mediated bactericidal activity of human antibodies to poly alpha 2-->8 N-acetylneuraminic acid, the capsular polysaccharide of Neisseria meningitidis serogroup B. J. Infect. Dis. 172: 1279- 1289). *N. meningitidis* strain CU385, with serologic classification B:4:P1.19,15, was used for the assay. The titers of bactericidal antibodies were expressed as the reciprocal of the greater dilution of antibodies evaluated, able to kill at least 50% of the bacteria with respect to controls. Table 4 shows the results.

**Table 4 Evaluation of bactericidal activity in sera of mice immunized with phages displaying peptides selected by panning the peptide library with anti-polysaccharide B monoclonal antibody. Mixtures of sera from mice inoculated with each immunogen were evaluated against N. meningitidis serogroup B strain CU385. SBT: Serum bactericidal titles. w/oT: without titer.**

| Groups | Immunogen | SBT |
|---|---|---|
| 1 | Phage NMGBPS 1 | 32 |
| 2 | Phage NMGBPS 2 | 8 |
| 3 | Phage NMGBPS 3 | 8 |
| 4 | Phage NMGBPS 4 | 16 |
| 5 | Control phage | w/oT |
| 6 | pPSC-B/HSA | 256 |

### Example 14. Mapping of peptides regions recognized by the anti-polysaccharide B monoclonal antibody and by the murine sera.

To study of the regions of peptides NMGBPS 1-4, recognized by the anti-polysaccharide B monoclonal antibody and by the sera from mice immunized with filamentous phages displaying these peptides, collections of five-residues peptide fragments overlapping by four residues and covering the entire sequences of peptides NMGBPS 1-4 were synthesized on cellulose membranes, were synthetized also fragments of these peptides containing successive deletions of aminoacid residues 1) from the amino terminal end, 2) from the carboxyl terminal end, and 3) simultaneously from both ends.

Figure 6 shows the design of peptide fragments synthesized for the mapping of the regions of peptide NMGBPS1. Figure 7 (left panel) shows the results obtained when the cellulose membrane was incubated with the anti-polysaccharide B monoclonal antibody. Among the 5-aminoacid-residue peptides, those with sequences VWYVE and WYVEG were recognized. This result and the analysis of the pattern of reactivity obtained with the rest of the peptides allows to conclude that the minimum sequence within peptide NMGBPS1, necessary for the binding of the anti-polysaccharide B monoclonal antibody is the tetrapeptide WYVE. Figure 7 (right panel) shows that the incubation of the peptide collection synthesized on paper with a mixture of sera from the mice immunized with peptide NMGBPS1 exposed on filamentous phages, rendered recognition of the peptide fragment with sequence WYVEG, which is consistent with the results obtained for the monoclonal antibody. The mixture of sera recognized also the points corresponding to peptide fragments of sequence RPPVW and PPVWY, which indicated the formation of a second epitope in peptide NMGBPS1, able to induce the production of antibodies. From structure prediction of peptide NMGBPS1 it was concluded that for the formation of this second epitope it is fundamental the presence of the two P, since they introduce turns into the structure of the peptide that would favour the presentation of this region of the peptide to the immune system.

### Example 15. Synthesis of peptide collections derived from point mutations to peptides NMGBPS1-4, and reactivity of said peptides with the anti-polysaccharide B monoclonal antibody.

Taking into consideration the results derived from the experiments explained in Example 14 we considered the search of variants of peptides NMGBPS1-4 that 1) did not contain any additional regions to the region of binding to the anti-polysaccharide B monoclonal antibody, with the capacity of induction of immune response and/or 2) that had greater affinity with the anti-polysaccharide B monoclonal antibody. To achieve this we selected the methodology of synthesis of a peptide collection in which each the P of the peptide (positions 7 and 8) were replaced by A, and 1 to 3 point changes with respect to the original aminoacids were introduced in the positions corresponding to the sequence necessary for the binding of the anti-polysaccharide B monoclonal antibody. The original aminoacids were changed by others considering such criteria as charge, hydrophoficity and hydrofilicity, polarity, structural homology and the volume of the aminoacids lateral residues. Also, peptides were synthesized in which in each position one A was introduced.

Figure 8 shows the peptide collection synthesized for the optimization of peptide NMGBPS1. Peptide 1 corresponds with the NMGBPS1 sequence. In all peptides the Pro at positions 7 and 8 were simultaneously replaced by Ala Ala. In the positions corresponding to the amino acids necessary for the binding of the anti-polysaccharide B monoclonal antibody, were introduced one to three point changes, taking into account the criteria explained in the previous paragraph. Figure 9 shows the reactivity of the peptide collection shown in Figure 8 with the anti-polysaccharide B monoclonal antibody. The simultaneous substitution of P₇ by A₇ and P₈ by A₈ (peptide 7, Figure 9) decreased the affinity of binding of the peptide NMGBPS1 (peptide 1, Figure 9) with the antibody anti-polysaccharide B. Taking this into account, in the analysis of the reactivity of the anti-polysaccharide B monoclonal antibody with other synthetic peptides, the peptide conserving intact the epitope necessary for the binding of the monoclonal antibody, but with Ala in each 7 and 8 position was chosen as the positive control (peptide 7, Figure 9). This allowed us to identify position 12, with V in the original peptide as an advantageous position for the introduction of changes. For example, the change of V₁₂ by A₁₂ (peptide 5, Figure 10) increased the affinity with the monoclonal antibody with respect to the positive control (peptide 7, Figure 10), whereas the substitution of V₁₂ by S₁₂ (peptide 11, Figure 10) or V₁₂ by E₁₂ (peptide 12, Figure 10), generate peptides conserving more than 85% of the reactivity with the monoclonal antibody, with respect to the positive control (peptide 7, Figure 10). In the peptides with the changes V₉ by A₉ (peptide 16, Figure 10), and Y₁₁ by E₁₁ (peptide 14, Figure 10) or and Y₁₁ by I₁₁ (peptide 2, Figure 10) more than 75% of the reactivity to the anti-polysaccharide B monoclonal antibody with respect to the positive control was conserved (peptide 7, Figure 10). Changes in other positions lowered peptide reactivity with the monoclonal antibody below 75% with respect to the positive control. The sequences corresponding to peptides shown in Figure 8 as 2, 5, 7, 11, 12, 14 and 16 are in the list of sequences as Seq. ID No. No. 9, Seq. ID No. No. 10, Seq. ID No. No. 11, Seq. ID No. No.12, Seq. ID No.13, Seq. ID No. 14 and Seq. ID No. No. 15.

Figure 11 Shows the peptides with substitutions made in the position 13. It should be noted that this position, with an E in the original peptide, is not favorable for the introduction of mutation, since replacing this amino acid the reactivity with the monoclonal antibody is very low in comparison with the reactivity with the positive control (peptide 7, Figure 11). The introduction of other changes in other aminoacids positions did not improve the reactivity with the antibody when amino acid 13 is changed. Therefore the glutamic acid present in the peptide sequence is essential for recognition.

Considering the previous results, a new collection of peptides was synthesized, with only one of the two P replaced by A and as simultaneous substitutions those preserving more than 85% of reactivity with anti-polysaccharide B antibody with respect to the positive control peptide.

Figure 12 shows the second peptide collection that was synthesized to continue the optimization of the sequence of peptide NMGBPS1. Sequence 1 corresponds with peptide NMGBPS1. We synthesized peptides in which the sequence for the binding of the anti-polysaccharide B antibody contained only the changes V₁₂ by A₁₂, V₁₂ by S₁₂ and V₁₂ by E₁₂ and containing as well changes in some of the P by A. As controls of the experiment were synthesized similar peptides containing all of the P of the original peptide or all the P changed simultaneously by A. Figure 13 shows the reactivity of the peptide collection shown in Figure 12 with the anti-polysaccharide B monoclonal antibody. The substitution of P₇ by A₇ allowed recovering of 97% reactivity with the antibody when the epitope binding to anti-polysaccharide B antibody was conserved intact and 81% of reactivity when the additional change V₁₂ by A₁₂ was introduced with respect to the peptide NMGBPS1. The sequences corresponding to the peptides shown in Figure 8 as 2-12 are in the list of sequences as Seq. ID No. 16, Seq. ID No. 17, Seq. ID No. 18, Seq. ID No. 19, Seq. ID No. 20, Seq. ID No. 21, Seq. ID No. 22, Seq. ID No. 23, Seq. ID No. 24, 25 Seq. ID No. and Seq. ID No. 26.

In another experiment the reactivity of peptides with amino acid W replaced by F was evaluated. Figure 14 shows the reactivity of the anti-polysaccharide B monoclonal antibody with peptides derived from NMGBPS1, with W₁₀ changed to F₁₀, and with the additional changes P₇ by A₇ or P₈ by A₈. Figure 10 are shows the sequences of the synthetic peptides. A 86% of the reactivity with the antibody, with respect to peptide NMGBPS1, was conserved in the peptide where the changes P₇ by A₇ and W₁₀ by F₁₀ were simultaneously introduced (peptide 2, Figure 14). The sequences corresponding to peptides shown in the foot of bars 2, 3 and 4 in Figure 14 are shown in the list of sequences as Seq. ID No. 27, Seq. ID No. 28 and Seq. ID No. 29.

### Example 16. Synthesis of peptides containing several copies of the minimal sequence necessary for binding the anti-polysaccharide B monoclonal antibody to peptides NMGBPS1-4.

Other strategy followed to define peptides with greater affinity to the anti-polysaccharide B monoclonal antibody was the synthesis of peptides containing several copies, in tandem, of the minimal sequence necessary for binding of the anti-polysaccharide B monoclonal antibody to peptides NMGBPS1-4. This strategy, additionally, would limit the immune response only against the region necessary for binding the anti-polysaccharide B monoclonal antibody in peptides NMGBPS1-4.

The synthetic peptides were evaluated by ELISA, in comparison with the original peptides, for inhibition of binding of the anti-polysaccharide B monoclonal antibody with meningococcus polysaccharide B. Polystyrene plates were coated with a solution of bacterial polysaccharide B. Mixtures of the anti-polysaccharide B monoclonal antibody, at a fixed concentration (5ug/ml), with different concentrations of the peptides, were added to the ELISA plates. Next, immunoglobulins specific for mice IgG conjugated to the enzyme horseradish peroxidase were added and the reactivity was revealed with a solution containing H₂O₂ and the chromogen o-phenylene-diamine (OPD). The reaction was stopped with sulfuric acid 2.5 N and A₄₉₂ₙₘ was read in an ELISA plate reader.

Figure 15 shows the results obtained with the synthesized peptide containing three copies of the minimal sequence necessary for binding of anti-polysaccharide B monoclonal antibody to the peptide NMGBPS1. This peptide was able to inhibit the binding of the anti-polysaccharide B monoclonal antibody to polysaccharide B more effectively than peptide NMGBPS1.

## Claims

1. Peptides denominated NMGBPS 1-25, **characterized by** being antigens able to generate in the receiving organism an immune response directed against carbohydrates with the chemical structure of repeated units of (α 2-8) linked N-replaced neuraminic acid and having the sequences of amino acids identified in the list of sequences as Seq. ID No.5 - Seq. ID No.29.

2. Peptides denominated NMGBPS1, NMGBPS2, NMGBPS3 and NMGBPS4, according to the claim 1, **characterized by** been encoded by the DNA fragments named ADN-NMGBPS1, ADN-NMGBPS2, ADN-NMGBPS3 and ADN-NMGBPS4, identified in the list of sequences as Seq. ID No.1, Seq. ID No.2, Seq. ID No.3 and Seq. ID No.4.

3. DNA fragments named ADN-NMGBPS1, ADN-NMGBPS2, ADN-NMGBPS3 and ADN-NMGBPS4, **characterized by** having the sequences of bases identified in the list of sequences as Seq. ID No.1, Seq. ID No.2, Seq. ID No.3 and Seq. ID No.4 and codify for peptides NMGBPS1-4 **characterized** for having the aminoacids sequences identified in the list of sequences as Seq. ID No.5, Seq. ID No.6, Seq. ID No.7 and Seq. ID No.8.

4. Peptides denominated NMGBPS 1-25 or fragments of these, **characterized by** being the active component of a pharmaceutical formulation, able to generate in the receiving organism an immune response directed against carbohydrates with the chemical structure of repeated units of (α 2-8) linked N-replaced neuraminic acid, according to the claim 1.

5. Pharmaceutical formulation according to the claim 4, **characterized by** containing polysaccharide antigens.

6. Pharmaceutical formulation according to the claims 4 and 5, **characterized by** one of the components of the formulation is a capsular polysaccharide from *N. meningitidis.*

7. Pharmaceutical formulation according to the claims 4, 5 and 6, **characterized by** containing a protein-polysaccharide conjugate.

8. Pharmaceutical formulation according to the claims 4, 5, 6 and 7 **characterized by** it is a formulation to be administered by parenteral, mucosal or oral route.

9. Pharmaceutical formulation according to the claims 4, 5, 6, 7 and 8 **characterized by** containing mimetics structures or mimotopes of at least one of the peptides NMGBPS 1 - 25.

10. Peptides denominated NMGBPS Seq. ID No.s 5 - 8, 16 - 26 and 27 - 29, according to the claims 1 and 8, **characterized by** the Proline are replaced by Alanine.

11. Peptides denominated NMGBPS Seq. ID No.s 7 - 26 according to the claims 1 and 9, **characterized by** the Tryptophan are replaced by Phenylalanine.

12. Peptides denominated NMGBPS Seq. ID Nos. 5 - 6, 8 - 29, according to the claims 1 and 9, **characterized by** the Valine are replaced by Alanine.

13. Peptides denominated NMGBPS Seq. ID Nos. 5 - 6, 8 - 29, according to the claims 1 and 9, **characterized by** the Valine are replaced by Serine.

14. Peptides denominated NMGBPS Seq. ID Nos. 5 - 6, 8 - 29, according to the claims 1 and 9, **characterized by** the Valine are replaced by Glutamic Acid.

15. Peptides denominated NMGBPS Seq. ID Nos. 5, 9-13 and 16-29 according to the claims 1 and 9, **characterized by** the Tyrosine is replaced by Glutamic Acid.

16. Peptides denominated NMGBPS Seq. ID Nos. 5, 9-13 and 16-29 according to the claims 1 and 9, **characterized by** the Tyrosine is replaced by Isoleucine.

17. Peptides denominated NMGBPS 1-25, according to the claims 1 and 9, **characterized by** any of the Glutamic Acid of the peptide sequences is essential for the recognition of protective antibodies against *the Neisseria meningitidis,* induced by them.

18. Pharmaceutical formulation **characterized by** containing at least one of the peptides or at least one of the proteins of the claims 4, 5, 6, 7, 8 and 9, used as carriers of antigens of diverse nature.

19. Peptides denominated NMGBPS 1-25 according to the claims 1, or fragments of these, **characterized by** being a component for the diagnosis of diseases in which the target of the immune response are carbohydrates with the chemical structure of repeated units of (α 2-8) linked N-replaced neuraminic acid.
